# EUROPEAN PATENT APPLICATION

(11) **EP 1 279 737 A1**
(43) Date of publication of application: **29.01.2003**
(21) Application number: 01202878.3
(22) Date of filing: 27.07.2001
(51) Int. Cl.: C12N 15/82, C12N 15/74, C12N 5/10, A01H 5/00, C12N 9/10, C12N 15/11

(54) **Transformation method for obtaining marker-free plants**

(71) Applicant: Coöperative Verkoop- en Productievereniging, van Aardappelmeel en Derivaten Avebe B.A., 9641 JA Veedam (NL)
(72) Inventor: Wolters, Anna M. A., 6708 RE Wageningen (NL); Heeres, Paul, 7876 CG Valthermond (NL); Visser, Richard G. F., 6721 ET Bennekom (NL); de Vetten, Nicolaas C. M. H., 9713 DE Groningen (NL); van der Meer, Ingrid M., 6703 AV Wageningen (NL)
(74) Representative: Prins, Adrianus Willem

(57) **Abstract**

The invention relates to a transformation method for obtaining transgenic plants and plants obtained with said method. The invention provides a method for transforming a plant cell comprising providing a plant cell with a recombinant nucleic acid comprising a T-DNA construct allowing for transfer of said construct into the genome of a plant cell, said construct provided with a foreign nucleic acid that is free of nucleic acid encoding a selective marker.

## Description

Transformation of plants using transforming bacteria such as Agrobacterium spp. to obtain transgenic plants expressing a heterologous gene or gene fragment of interest has been known since the late seventies and early eighties of the last century when it was found that the Ti (tumor inducing) plasmid of said bacterium could be used as a vector in genetic engineering of plants. The wild-type plasmid induces plant cells to produce tumor cells, but it can be modified so that it can carry foreign gene constructs into cells without necessarily making the recipient cells tumorous. During tumor induction, a specific segment of the Ti plasmid, called the T-DNA (transferred DNA), integrates into the host plant nuclear DNA. In genetic engineering of plants, said T-DNA is modified and now carries the foreign gene construct to be integrated in the plant's DNA so that a transgenic plant can be obtained.

A transformation procedure for obtaining transgenic plants generally consists of infection of a plant cell with a transforming bacterium, which generally comprises an essentially non-tumorigenic Agrobacterium strain, which bacterium is provided with a recombinant nucleic acid comprising a T-DNA vector construct allowing for transfer of said construct into the genome of a plant cell, said construct essentially comprising the desired nucleic acid, gene or gene fragment that one wishes to see expressed in a finally transformed plant and a selective marker nucleic acid or selection gene. This desired heterologous gene and the marker are in general located on a plasmid or vector in a piece of the T-DNA, which is the DNA flanked by at least one, or located between two imperfect direct repeats of most often 24 basepairs length, called the T-DNA borders. Transfer of the heterologous gene/selection gene construct into the plant cell takes place in a process whereby vir genes (located on the same or different plasmid) are involved to accommodate transfer and integration of the T-DNA/gene construct. Vir-proteins (D1 and D2) cause nicking of the border repeats at a precise site whereby the T-DNA construct is cut at the T-DNA borders from the plasmid and inserted into the plant genome.

By growing the thus treated plant cells in an appropriate environment, whole plants can be regenerated, some of which carry the desired gene. To select for the desired transformed cells in the background of untransformed cells DNA encoding said selection gene and means for its expression is generally physically linked to the gene of interest on the T-DNA to permit the recovery of transformants. The presence of such a selection or marker sequence in the T-DNA is generally seen as an absolute requirement for efficient recovery; otherwise tenths- to hundreds-of-thousands putative transformants would need to be screened for the presence and functional insertion of the desired heterologous gene. Instead, putative transformants are always grown in a selective medium or under selective pressure appropriate for the selective marker chosen to give the transformed cells a selective advantage over the, initially over-abundantly present, non-transformed cells.

Among the selective markers or selection genes that are most widely used in plant transformation are the bacterial neomycin phosphotransferase genes (nptI, nptII and nptIII genes) conferring resistance to the selective agent kanamycin, suggested in EP131623 and the bacterial aphIV gene suggested in EP186425 conferring resistance to hygromycin. EP 275957 discloses the use of an acetyl transferase gene from *Streptomyces viridochromogenes* that confers resistance to the herbicide phosphinotricin. Plant genes conferring relative resistance to the herbicide glyphosate are suggested in EP218571. The resistance is based on the expression of a gene encoding 5-enolshikimate-3-phosphate synthase (EPSPS) that is relatively tolerant to N-phosphomethylglycine. Certain amino acids such as lysine, threonine, or the lysine derivative amino ethyl cysteine (AEC) and tryptophan analogs like 5-methyl tryptophan can also be used as selective agents due to their ability to inhibit cell growth when applied at high concentration. In this selection system expression of the selectable marker gene results in overproduction of amino acids by transgenic cells which permits the transgenic to grow under selection. Other selection marker genes encode an enzyme that after expression converts an encryptic carbon source into a carbon source that supports growth and proliferation of the transformed plant cells under conditions that contains minimal nutrients and in which the carbon source is replaced by an encryptic or latent carbon source that can not be utilized by non-transformed cells. An example of such a selection marker is the phosphomannose isomerase that converts non-utilizable mannose-6-phosphate into fructose-6-phosphate that can be used by plant cells as a carbon source (suggested in US6143562) or xylose isomerase from *Streptomyces rubiginosus* (Haldrup A., et al. 1998, Plant Cell Report 18:76-81).

However, the presence of antibiotic resistance genes and other selective markers sequences in the final transgenic plant obtained is in most cases considered undesirable. These sequences, albeit thought to be necessary for the transformation processes, do in general not positively contribute to the final transformed plant and in fact lessen its desirability to the consumer for a number of reasons. Consumer groups express concern about the widespread distribution of resistance markers in food products referring to a theoretical risk of a horizontal transfer of transgene selection genes into gut bacteria. Environmental groups are concerned about the risk of cross-pollination between the transgenic plant and related species which can lead to a transfer of resistance traits into weeds, jeopardizing the long-term use of transgenic crops and causing potential ecological problems. Consequently, generating marker-free plants would likely alleviate the public weariness towards acceptance of transgenic crops.

A number of systems have so far been developed to facilitate the removal of selective marker genes. Co-transformation of two different constructs can result in transgenic lines that have integrated both transgenes and after a genetic cross the selective marker can be segregated away from the gene of interest. This system requires however that the marker gene is located at an unlinked location meaning screening of many more independent transformation events. Moreover, many cross pollinating and in particular vegetatively propagated crops, and especially tuberous crops like potato and cassave, are highly heterozygous and removal of the marker sequence by genetic segregation would require many years to find a clone with suitable field performance. Several transposable element systems and site-specific recombination systems have been employed for marker removal (Sugita K, et al. 2000 Plant J 22: 461-469; Zuo J, et al. 2001 Nature Biotech 19: 157-161). These systems require the expression of a transposase or recombinase that mediates the deletion of regions bracketed between recombination or transposase target sequences, and the subsequent removal of the marker gene by genetic segregation. Also these systems are time consuming as well as impractical for species that are mainly propagated vegetatively Moreover, deleted fragments can reinsert into other genomic positions. Another approach to induce DNA deletions is based on intrachromosomal homologous recombination between two homologous sequences. Intrachromosomal homologous recombination frequencies are often too low for an efficient application of this system to produce deletions of transgene regions. Using the attP region of bacteriophage λ this recombination frequency could be increased (Zubko E, et al. 2000 Nature Biotech 18: 442-445). However, the process is still unpredictable and too inefficient to generate hundreds to thousands of independent transformation events to find the clone with suitable agronomical performance.

The invention provides a method for producing transgenic plants, in particular of cross pollinated and vegetatively propagated crops, that contain a gene of interest and that are essentially free of other foreign or heterologous ancillary nucleic acids such as selective marker genes. All of the above mentioned systems for the generation of marker-free transformants are employed on the assumption that isolation of transformants without a selective marker is practically unfeasible, at least with transformation protocols using T-DNA constructs or corresponding transforming bacteria. The method provided herein allows for the production of plants which contain a desired gene, but which are essentially free of vector sequences and/or marker sequences used to transform the plant. Surprisingly, the invention provides a method for the transformation of a plant or plant cell comprising using a transforming bacterium, such as Agrobacterium spp., and its T-DNA to obtain transgenic plants expressing a desired heterologous gene of interest, wherein said T-DNA is provided with the desired gene or gene fragment but does not comprise an additional selection gene or fragment thereof.

For this purpose, the invention provides an isolated or recombinant nucleic acid comprising a T-DNA or functional equivalent thereof allowing for transfer of said T-DNA into the genome or nuclear DNA of a plant cell, said T-DNA provided with a nucleic acid that is free of a nucleic acid encoding a selective marker, and the use of such a T-DNA construct in genetic engineering of plants. Consequently, the method provided herein does not require growing putative transformants in selective medium or under selective pressure to let the truly transformed plant emerge. Instead, selection for the desired transformant can easily be achieved by testing for the presence of the desired heterologous gene or gene fragment or construct itself, for example by using commonly known nucleic acid techniques, such as detection by Polymerase Chain Reaction or by hybridisation with complementary sequences in routine Southern blotting experiments. Testing need to be done for the presence or absence of the recombinant T-DNA construct used for transfer of said construct into the DNA of the plant cell, especially for that part of the construct provided with the desired foreign nucleic acid that is free of nucleic acid encoding a selective marker. It is preferred that a nucleic acid according to the invention comprises a T-DNA construct which comprises at least one T-DNA border, but for ease of integration preferably said foreign nucleic acid is flanked by T-DNA border repeats or functional equivalents thereof. The VirD1 and VirD2 proteins of the Agrobacterium host cell recognize the border repeat sequences and produce a single stranded nick between the third and the fourth base in the bottom strand of each border repeat. These nicks determine the initiation termination sites of the T-strand at the right and left border, respectively. The left border was found to be non-essential for transfer of the T-DNA, but helps to define the left end of the T-DNA. The right border seems to be most essential in T-DNA transfer. Ti plasmids with the T-DNA right border region deleted are in general avirulent. (Holsters, M. et al. Plasmid 3, 212-230, 1980). Deletion of the left border region has no effect on virulence. The sequence context of the repeats determine their relative activity during T-DNA transfer ( Wang et al. Mol. Gen. Genet. 210, 338-346, 1980). A typical example of a border repeats of the pBIN19 plasmid is Right Border 5'-TGACAGGATATATTGGCGGGTAAAC-3' and Left Border 5'-TGGCAGGATATATTGTGGTGTAAAC-3'.Of course, practicing the invention is most easily achieved with a nucleic acid according to the invention wherein said T-DNA is derived from the Ti plasmid of an Agrobacterium spp, especially wherein said Agrobacterium comprises A. tumefaciens, said bacterium and expertise being most widely available.

In one embodiment, a nucleic acid according to the invention is provided wherein said foreign nucleic acid allows for regulation of the expression of a target gene in said genome. Both upregulation (or overexpression) and downregulation can be achieved by "sense" technology. If a full length copy of the target gene is inserted into the genome a range of phenotypes can be obtained, some overexpressing the target gene, some under-expressing. A population of plants produced by this method may then be screened and individual phenotypes isolated. A preferred embodiment comprises a method wherein said regulation comprises downregulation. The inhibition of expression of a target gene, commonly referred to as "gene silencing" can be achieved by "antisense downregulation" and "sense downregulation" (also, referred to as "cosuppression"). In antisense downregulation, a DNA fragment which is complementary to all or part of an endogenous target gene is inserted into the genome in reverse orientation. While the mechanism has not been fully elucidated, one theory is that transcription of such an antisense gene produces mRNA which is complementary in sequence to the mRNA product transcribed from the endogenous gene. The antisense mRNA then binds with the naturally produced "sense" mRNA to form a duplex which inhibits translation of the natural mRNA to protein. Antisense downregulation technology is well-established in the art and used routinely in laboratories around the world. Gene silencing can therefore be achieved by inserting into the genome of a target organism a copy of at least a fragment of the target gene coding sequence which copy may comprise either the whole or part or be a truncated sequence and may be in sense or antisense orientation. Additionally, intron sequences which can be obtained from the genomic gene sequence may be used in the construction of suppression vectors. There have also been reports of gene silencing being achieved within organisms of both the transgene and the endogenous gene where the only sequence identity is within the promoter regions.

In a much preferred embodiment, the invention provides a T-DNA construct wherein said foreign nucleic acid includes a inverted repeat of at least part of a polynucleotide region of said target gene. Although antisense and sense downregulation can result in complete silencing of the target gene, the efficiency is generally not very high. A maximum of 25% of the antisense transformants displayed complete silencing, while only about 10% of the transformants obtained with sense constructs showed some level of silencing (Smith et al. 2000 Nature 407: 319-320; Wolters and Visser, 2000 Plant Mol Biol 43: 377-386). Recently, it has been observed that the inhibition of a selected target gene within an organism is enhanced, when the gene silencing vector includes a inverted repeat of all or part of a polynucleotide region of the target gene.
The inverted repeat sequence may consist of a T-DNA with one promoter driving expression of a sense copy of the cDNA together with another promoter in front of an antisense copy of the same cDNA (Chuang and Meyerowitz, 2000 Proc Natl Acad Sci USA 97: 4985-4990) or the T-DNA may contain a cDNA sequence flanked on both ends by a promoter (LaCount et al. 2000 Mol Biochem Parasit 111: 67-76), or the T-DNA may contain a promoter driving transcription of an inverted repeat of (part of) the cDNA (Hamilton et al. 1998; Smith et al. 2000 Nature 407: 319-320; Wang and Waterhouse, 2000 Wang MB, Waterhouse PM (2000) Plant Mol Biol 43: 67-82) or the promoter (Mette et al. 2000 EMBO J 19: 5194-5201) of the gene to be silenced. Some inverted repeat constructs are reported to result in 100% of the transformants displaying silencing of the target gene (Smith et al. 2000 Nature 407: 319-320) in case of using an intron as a spacer between the repeats. The spacer fragment contributes to the stability of the perfect inverted repeat sequences, but is not required for the specificity of the silencing. In a particularly preferred embodiment of a nucleic acid according to the invention said target gene encodes a granule-bound starch synthase (GBSSI) enzyme. The detailed description herein gives a striking increase in the frequency of completely silenced transformants following the inclusion of a short repeated region of the target gene encoding the granule-bound starch synthase (GBSSI) enzyme of potato within a transgene.

Also is provided a nucleic acid according to the invention wherein said foreign nucleic acid allows for expression of a heterologous polypeptide in said plant cell.
Suitable polypeptides are manifold, typical examples of foreign nucleic acid or genes that are of interest to transfer marker-free into plants are those encoding for proteins and enzymes that modify metabolic and catabolic processes. Other examples are genes that may encode a protein giving added nutritional value to the plant as a food or crop. Typical examples include plant proteins that can inhibit the formation of anti-nutritive factors and plant proteins that have a more desirable amino acid composition (e.g. a higher lysine content than the non-transgenic plant). In a preferred embodiment, said nucleic acid or gene of interest encodes a polypeptide which comprises an enzyme. The gene of interest may code for an enzyme that can be used in food processing such as chymosin, thaumatin and alpha-galactosidase. The gene of interest may also code for an agent for introducing or increasing pathogen resistance. The gene of interest may code for a non-natural plant compound that is of benefit to animals or humans. For example, the gene of interest could code for a pharmaceutically active protein or enzyme such as any one of the therapeutic compounds insulin, interferon, human serum albumin, human growth factor and blood clotting factors. In this regard, the transformed cell or organism could prepare acceptable quantities of the desired compound which would be easily retrievable from, for example, the tubers. Preferably, the gene of interest is a gene encoding for a protein or peptide having a high nutritional value, a feedback-insensitive amino acid biosynthesis enzyme such as dihydrodipicolinate synthase (EC 4.2.1.52, DHPS), an enzyme or peptide conferring disease resistance, a sense or antisense transcript such as that for patatin, ADP-glucose pyrophosphorylase, alpha.-amylase, branching enzyme, granule-bound starch synthase, soluble starch synthases, a protease or a glucanase.
The invention also provides a vector or plasmid comprising a nucleic acid according to the invention, and host cells comprising such vectors or nucleic acid. A preferred host cell comprises an Agrobacterium. For practicing the method of transformation according to the invention it is even preferred to use a substantially virulent Agrobacterium such as A. tumefaciens, as exemplified by strain A281 or a strain derived thereof or another virulent strain available in the art. These Agrobacterium strains carry a DNA region originating from the virulence region of the Ti plasmid pTiBo542 containing the *virB, virC* and *virG* gees. The virulence (vir) gene products of *A. tumefaciens* coordinate the processing of the T-DNA and its transfer into plant cells. Vir gene expression is controlled by *virA* and *virG,* whereby *virA* upon perception of an inducing signals activates *virG* by phosphorylation. *VirG,* in turn, induces the expression of *virB,C,D,E.* These genes code for proteins involved in the transfer of DNA. The enhanced virulence of pTiBo542 is thought to be caused by a hypervirulent *virG* gene on this Ti plasmid (Chen et al. Mol. Gen. Genet 230: 302-309, 1991). However, besides by Agrobacterium infectium, there are other means to effectively deliver of DNA to recipient plant cells when one wishes to practice the invention. Suitable methods for delivering DNA to plant cells are believed to include virtually any method by which DNA can be introduced into a cell, such as by direct delivery of DNA such as by PEG-mediated transformation of protoplasts (Omirulleh et al., 1993), by desiccation/inhibition-mediated DNA uptake (Potrykus et al., Mol. Gen. Genet., 199:183-188, 1985), by electroporation (U.S. Pat. No. 5,384,253), by agitation with silicon carbide fibers (Kaeppler et al., 1990; U.S. Pat. No. 5,302,523; and U.S. Pat. No. 5,464,765), and by acceleration of DNA coated particles (U.S. Pat. No. 5,550,318; U.S. Pat. No. 5,538,877; and U.S. Pat. No. 5,538,880). Through the application of techniques such as these, certain cells from virtually any plant species may be stably transformed, and these cells developed into transgenic plants.
In Microprojectile Bombardment, particles may be coated with nucleic acids and delivered into cells by a propelling force. Exemplary particles include those comprised of tungsten, gold, platinum, and the like. It is contemplated that in some instances DNA precipitation onto metal particles would not be necessary for DNA delivery to a recipient cell using microprojectile bombardment. However, it is contemplated that particles may contain DNA rather than be coated with DNA.
For microprojectile bombardment transformation in accordance with the current invention, both physical and biological parameters may be optimized. Physical factors are those that involve manipulating the DNA/microprojectile precipitate or those that affect the flight and velocity of either the macro- or microprojectiles. Biological factors include all steps involved in manipulation of cells before and immediately after bombardment, such as the osmotic adjustment of target cells to help alleviate the trauma associated with bombardment, the orientation of an target tissue relative to the particle trajectory, and also the nature of the transforming DNA, such as linearized DNA or intact supercoiled plasmids. It is believed that pre-bombardment manipulations are especially important for successful transformation.
Accordingly, it is contemplated that one may wish to adjust various bombardment parameters in small scale studies to fully optimize the conditions. One may particularly wish to adjust physical parameters such as DNA concentration, gap distance, flight distance, tissue distance, and helium pressure. It is further contemplated that the grade of helium may effect transformation efficiency. One also may optimize the trauma reduction factors by modifying conditions which influence the physiological state of the recipient cells and which may therefore influence transformation and integration efficiencies. For example, the osmotic state, tissue hydration and the subculture stage or cell cycle of the recipient cells may be adjusted for optimum transformation.
The transforming DNA used for microprojectile transformation may contain an expression cassette comprising generally of a cDNA, gene or genes which one desires to introduce into the cells, and still further, may include a promoter and 3' region operatively linked to the exogenous gene. The optimized gene construct is described in present invention. The DNA segment may additionally include a second, third, fourth, fifth, sixth, or any additional number of exogenous genes capable of being placed on a single DNA molecule and transformed into a recipient cell.
In particular embodiments of the invention, the DNA segments will not include a transformation selectable or screenable marker gene. Generally one may employ a selectable or screenable marker gene in addition to, the expressible gene of interest in order to improve the ability to identify transformants. "Marker genes" are genes that impart a distinct phenotype to cells expressing the marker gene and thus allow such transformed cells to be distinguished from cells that do not have the marker. Such genes may encode either a selectable or screenable marker, depending on whether the marker confers a trait which one can "select" for by chemical means, i.e., through the use of a selective agent (e.g., a herbicide, antibiotic, amino acid analog or the like), or whether it is simply a trait that one can identify through observation or testing, i.e., by "screening" (e.g., the R-locus trait, beta.-glucuronidase or uidA gene ). For production of transformants free of vector DNA sequences it will be desirable to deliver DNA to cells that does not contain DNA sequences necessary for maintenance of the plasmid vector in the bacterial host, e.g., E. coli, such as antibiotic resistance genes, including but not limited to ampicillin, kanamycin, and tetracycline resistance, and prokaryotic origins of DNA replication. In such case, a DNA fragment containing the ransforming DNA may be purified prior to transformation. Purification can be achieved by for example gel electrophoresis on a agarose gel, followed by recovery from the agarose gel.
Control the copy number of introduced transgenes and an efficiently production of low-copy transformants can be achieved by end-modifying of nucleic acid segments by dephosphorylation or by blunting the ends of the nucleic acid segments (WO99/32642). The recipient plant cells for transformation with the microprojectile bombardment compositions of the invention may be from potentially any transformable monocot or dicot plant. Preferred monocot plant cells for use with the invention are from rice, wheat, barley, oats, rye, millet, sorghum, sugarcane, turfgrass and maize. Preferred dicot plant cells for use with the invention include cotton, tomato, citrus, tobacco, soybean and particularly potato and cassava. After effecting delivery of exogenous DNA to recipient cells, the next steps generally concern identifying the transformed cells for further culturing and plant regeneration. In the present invention cells after delivery of exogenous DNA will be cultured in media that supports regeneration of plants. After shoot development DNA or RNA detection methods are used for detecting plantlet containing one or more transgenes. The method includes the isolation of nucleic acid from plantlets or a pool of plantlets according to standard methodologies (Sambrook et al., 1989). The nucleic acid may be genomic DNA, RNA or mRNA. The specific nucleic acid of interest, being part of the transgene is identified in the sample directly using amplification. Next, the identified product is detected. In certain applications, the detection may be performed by visual means (e.g., ethidium bromide staining of a gel). Alternatively, the detection may involve indirect identification of the product via chemiluminescence, radioactive scintigraphy of radiolabel or fluorescent label or even via a system using electrical or thermal impulse signals. A variety of different assays are contemplated in the screening of transgenic plants created using the methods of the current invention. These techniques can be used to detect for both the presence of particular genes as well as rearrangements that may have occurred in the gene construct. The techniques include but are not limited to, polymerase chain reaction (PCR), fluorescent in situ hybridization (FISH), direct DNA sequencing, PFGE analysis, Southern or Northern blotting, single-stranded conformation analysis (SSCA), RNAse protection assay, allele-specific oligonucleotide (ASO), dot blot analysis, denaturing gradient gel electrophoresis, RFLP and PCR-SSCP or chip-based DNA technologies. The transformed plantlet, identified by mucleic acid detection methods, will then be allowed to mature into plants. After the regenerating plants have reached the stage of shoot and root development, they may be transferred to a greenhouse for further growth and testing.

Furthermore, the invention provides plant cell comprising a nucleic acid according to the invention, and regenerated plant, or part thereof, derived from such a plant cell. Particularly provided are tuberous plants, or parts thereof, which are preferably selected from the group of potato or cassave plants. Such a potato or cassave plant as provided herein comprises a so-called marker-free high-amylopectine tuber (for example potato or cassave), being essentially devoid of amylose due to downregulation of the gene encoding the granule-bound starch synthase enzyme. Such a potato or cassava plant contains an amylose content of less than 5%, does essentially not contain any transformation selection marker genes like antibiotic or herbicide resistance genes, these plants have not integrated in their genome vector sequences used for the transformation process, like the whole or parts of Agrobacterium Ti plasmid, and contain low copy nummer of the T-DNA insert, preferably one copy. Also provided is a high lysine plant (preferably potato or cassave) that contain more than 20% lysine of the total free amino acid content, or more than 15% methionine of the total free amino acid content, or more than 30% amylose or more than 2% coagulated protein in combination with essentially not containing transformation selection marker genes like antibiotic or herbicide resistance genes, not having integrated in their genome vector sequences used for the transformation process, like the entire or parts of Agrobacterium Ti plasmid, and containing low copy numbers of the T-DNA insert, preferably one copy.

Furthermore, the invention provides a method for transforming a plant cell comprising providing a plant cell with a nucleic acid according to the invention. In one embodiment, the invention provides a method for the production and isolation of transformants showing silencing of an endogenous gene or overexpression of an endogenous gene, without the involvement of a selective marker gene. We have shown herein that with this method it is even possible to obtain essentially marker gene-free, backbone vector DNA-free transformants, having advantageously only 1 insert of the T-DNA inserted, in which an endogenous gene is silenced or overexpressed. In another embodiment, a method is provided for the production and isolation of transformants wherein a heterologous gene is expressed, said transformants likewise being essentially marker-free and backbone vector DNA-free. As said, a method is provided wherein it is not necessary to culture the truly transformed cell under selective pressure derived from a selective medium to outcompete untransformed cells, the efficiency of transformation using a method according to the invention is so high that such selective culturing is no longer required, allowing to test for the inserted nucleic acid itself immediately, a routine which is most often practiced anyway. All that is required is testing at least a part of the progeny of said cell for the presence or absence of at least a functional part of a nucleic acid according to the invention. Such progeny can consist of parts of roots or shoots, or of individual cells, thereby leaving sufficient transformed material for further regeneration and vegetative propagation. It is preferred to select transformants such as transformed shoots through the use of nucleic acid detection such as Polymerase Chain Reaction procedures and / or phenotypic screening. Transformants that carry the gene of interest and are free of ancillary nucleic acids may also be detected by Southern hybridization, Polymerase Chain Reaction procedures and other detection methods available in the art. Such testing may further comprise testing at least a part of the progeny of said cell for the presence or absence of undesired vector nucleic acid. In short, this invention provides methods for producing transgenic plants that contain a gene of interest and that are essentially free of ancillary unwanted nucleic acids. In a preferred embodiment the invention provides for transforming plants using an optimized transformation procedure using an (preferably virulent) Agrobacterium strain, an optimized gene construct for efficient inhibition or overexpression of a gene and an efficient method to detect transformants biochemically instead of due to selective pressure, for example, by Southern blot hybridization, Polymerase Chain Reaction procedures or other nucleic acid detection methods available.

### Legends

- Figure 1.: Map of the GBSS cDNA antisense gene silencing vector
- Figure 2: Map of the plasmid PMTL1.1 containing the 1.1 kb 5' end of the GBSS cDNA used in the preparation of the plasmids of Figure 4, 5, 6, 7, 8, 13, 14, and 15.
- Figure 3: Map of the plasmid pMTL1.3 containing the 1.3 kb 3' end of the GBSS cDNA used in the preparation of the plasmids of Figure 4, 5, 6, 7, 8, 13, 14, and 15.
- Figure 4.: GBSSI gene silencing vector pKGBA50-IR1.3 with nptII selection marker gene containing the 1.3 kb 3' end of the GBSS cDNA in an inverted repeat configuration interrupted by the 1.1 kb 5' end of the GBSS cDNA.
- Figure 5.: GBSSI gene silencing vector pKGBA50-DR1.3 with nptII selection marker gene containing the 1.3 kb 3' end of the GBSS cDNA in a tandem repeat configuration interrupted by the 1.1 kb 5' end of the GBSS cDNA.
- Figure 6.: GBSSI gene silencing vector pKGBA50-IR1.1 with nptII selection marker gene containing the 1.1 kb 5' end of the GBSS cDNA in an inverted repeat configuration interrupted by the 1.3 kb 3' end of the GBSS cDNA.
- Figure 7.: GBSSI gene silencing vector pKGBA50-DR1.1 with nptII selection marker gene containing the 1.1 kb 5' end of the GBSS cDNA in a tandem repeat configuration interrupted by the 1.3 kb 3' end of the GBSS cDNA.
- Figure 8.: Selection marker-free GBSSI gene silencing vector pKGBA50mf-IR1.1 containing the 1.1 kb 5' end of the GBSS cDNA in an inverted repeat configuration interrupted by the 1.3 kb 3' end of the GBSS cDNA.
- Figure 9.: Results of transformation of potato plants cv. Karnico with 4 different GBSSI gene silencing constructs containing the GBSSI cDNA in an inverted or tandem repeat configuration comparing to the traditional GBSSI antisense construct. After selection of transformants tuber induction media was added to in vitro grown plants. After 2 to 6 weeks microtubers had developed on most shoots.
Starch from cut tuber surfaces was stained with iodine. Transformants having granules staining blue with iodine solution are non-silenced (none), tubers staining red are silenced (completely/strong) and "weak or medium" indicate partially silenced transformants.
- Figure 10.: Map of plasmid pAAP105 mf containing feedback-insensitive potato DHDPS cDNA in control of the GBSSI promoter without plant transformation selection markers.
- Figure 11.: Free lysine concentration (% of total free amino acids) of microtubers derived from different independent transformants or control plants. Tubers were induced by adding Tuber induction media to in vitro grown plants. After 2 to 6 weeks microtubers had developed on most shoots. Tissue (0.5-1.0 gram) was homogenised in 2 ml Pi-buffer containing 1 mM dithiothreitol. Nor-leucine was added as an internal standard. Free amino acids were partly purified by extraction with 5 ml of a
water:chloroform:methanol mixture (3:5:12). After concentration by lyophilisation to 3 ml, a 20 microl sample was analysed by HPLC using a cation-exchange column (BIOCHROM 20, Amersham Pharmacia biotech).
- Figure 12.: Map of the plasmid pAAP172 containing the 1.1 kb feedback-insentive potato DHPS* cDNA on control of the potato GBSSI promoter used in the preparation of the plasmids of Figure 13, 14 and 15.
- Figure 13.: Construction of GBSSI gene silencing vector pKGBA50mf-R1.1 containing the feedback-insensitive potato DHPS* cDNA of pAAP105.
- Figure 14.: T-DNA of pDHPS-IR1.1 (tandem) containing the 1.1 kb 5' end of the GBSS cDNA in an inverted repeat configuration interrupted by the 1.3 kb 3' end of the GBSS cDNA, and the feedback-insensitive potato DHPS* cDNA in a tandem orientation without plant transformation selection markers.
- Figure 15.: T-DNA of pDHPS-IR1.1 (tandem) containing the 1.1 kb 5' end of the GBSS cDNA in an inverted repeat configuration interrupted by the 1.3 kb 3' end of the GBSS cDNA, and the feedback-insensitive potato DHPS* cDNA in a reverse orientation without plant transformation selection markers.

### Detailed description

### Optimizing Plant transformation

High-throughput production of marker-free transformants as suggested in this invention calls for high transformation frequencies independent of the genotype. Optimizing transformation protocols for mono- and dicotyledonous plants has been the subject of many studies._For transformation of tuberous plants such as potato, several methods have been published. In many of these protocols the transformation frequencies are very genotype dependent. The essential differences between the different protocols is whether auxins are present during the whole regeneration period or whether a callus initiation phase is followed by a period of absence of auxin but presence of gibberellins. There are also differences between the published protocols in the composition of the media and in the precise levels and types of auxins and cytokinines (Hulme et al. 1992 Plant Molec. Biology 31, 161-167). A number of investigators have shown that ethylene accumulation can inhibit regeneration of potato plants and that this inhibition can be overcome by the presence of ethylene biosynthesis or ethylene action inhibitors (Perl et al. 1988 Plant Cell Rep. 7, 403-406). Accordingly, some protocols add silverthiosulphate (STS) in the regeneration medium. Other modifications can be added to the transformation protocol to increase transformation efficiency. For example, WO 0034491 suggested that reducing moisture conditions to reduce explant weight after Agrobacterium inoculation enhances the transformation efficiency. Possible methods to reduce the weight of the explants during co-culture include increasing the osmotic potential of the medium, addition of desiccants, including calcium oxide or sulphuric acid or air-drying the explants.

For a commercial transformation process it is however convenient to have a single protocol that is efficient for a great range of cultivars. Comparing a number of different protocols on the regeneration of a range of potato cultivars Hulme et al. (1992 Plant Molec. Biology 31, 161-167) conclude that the protocol of Hovenkamp-Hermelink et al. (1988 Euphytica 39, 213-219) is a superior method to those published so far for a number of potato cultivars. We demonstrate herein that two transformation protocols for regeneration have a significant effect on the number of marker-free transformants obtained.

### Optimizing transformation efficiency

A preferred Agrobacterium strain utilized in a method of the invention is modified to contain a gene or genes of interest, or a nucleic acid to be expressed in the transformed cells. The nucleic acid to be transferred is incorporated into the T-region and is flanked by at least one T-DNA border sequence. A variety of Agrobacterium species are known in the art particularly for dicotyledon transformation including *Agrobacterium tumefaciens* and *Agrobacterium rhizogenes.* See, for example, Hooykaas, P.J. 1989 Plant Mol. Siol. 13:327; Smith et al. 1995 Crop Science 35:301; Chilton, M.O. 1993 Proc. Natl. Acad.Sci. USA 90:3119; 19:148; Ishida et al. 1996 Nature Biotechnol. 14:745; Komari, T. et al. 1996 The Plant Journal 10:165. In the Ti plasmid, the T-region is distinct from the vir region whose functions are responsible for transfer and integration. Binary vector systems have been developed where the manipulated T-DNA carrying foreign DNA and the vir functions are present on separate plasmids. In this manner, a modified T-DNA region comprising foreign DNA (the desired nucleic acid to be transferred) is constructed in a small plasmid which replicates in E. coli. This plasmid is transferred conjugatively in a tri-parental mating or by electroporation or by freeze-thaw procedure into *A. tumefaciens* which contains a compatible plasmid carrying virulence genes. The vir functions are supplied in trans to transfer the T-DNA into the plant genome. The transformation efficiency is largely influenced by the existence of a super-virulent vir region. Preferred is an *Agrobacterium tumefaciens* strain of the agropine, binary type. Especially preferred is the publicly available *Agrobacterium tumefaciens* strain EHA101, AGLO or AGL1. These strains contain a C58 bacterial chromosome and a disarmed derivative of the Ti plasmid referred to in the literature as TiBO542 originating from a strongly virulent Agrobacterium tumefaciens A281. [See, e.g., Hood E.E. et al, (1986) J. Bacteriology 168:1291-1301, Lazo G.R. et al. (1991) Biotechnology 9: 963-967]. Alternatively, an Agrobacterium strain can be used containing a plasmid equiped for enhanced virulence. Preferred is a superbinary vector to practice the invention with . Such a super-binary vector has been constructed containing a DNA region originating from the virulence region of Ti plasmid pTiBo542 (Jin et al. (1987) J. Bacteriol 169:4417-4425) contained in a super-virulent Agrobacterium tumefaciens A281 exhibiting extremely high transformation efficiency (Hood et al. (1984) Biotechnol. 2:702-709; Hood et al. (1986) J. Bacterial. 168:1283-1290; Komari et al. (1986) J. Bacteriol. 166:88-94; Jin et al. (1987) J. Bacterial. 169:4417-4425; Komari T. (1989) Plant Science 60:223-229; ATCC Accession No. 37394).

Super-binary vectors include pTOK162 (Japanese Patent Appl. (Kokai) No. 4222527, EP-A-504,869, EP-A-604,662, and U.S. Patent No. 5,591,616) and pTOK233 (Komari, T. (1990) Plant Cell Reports 9:303-306; and Ishida et al. (1996) Nature Biotechnology 14:745). Super-binary vector pTOK162 is capable of replication in both *E. coli* and in *A. tumefaciens.* Additionally, the vector contains the virB, virC, and virG genes from the virulence region of pTiBo542. The plasmids were introduced into the Agrobacterium strains by triple cross (Ditta G. et al., 1980; Proc. Natl. Acad. Sci. USA 77: 7347-7351). Besides the type of Agrobacterium strain, it is known that supplementation of the Agrobacterium suspension with a phenolic compound such as acetosyringone can enhance the transformation efficiency (see Townsend, J. A. et al., US 5,563,055). Typical concentration ranges have been in the range of 100 to 200 microM.

### Optimizing the gene construct

The inhibition of expression of a target gene, commonly referred to as "gene silencing" can be achieved by "antisense downregulation" and "sense downregulation" (also, referred to as "cosuppression"). In antisense downregulation, a DNA fragment which is complementary to all or part of an endogenous target gene is inserted into the genome in reverse orientation. While the mechanism has not been fully elucidated, one theory is that transcription of such an antisense gene produces mRNA which is complementary in sequence to the mRNA product transcribed from the endogenous gene. The antisense mRNA then binds with the naturally produced "sense" mRNA to form a duplex which inhibits translation of the natural mRNA to protein.

Antisense downregulation technology is well-established in the art and used routinely in laboratories around the world. Both overexpression and downregulation are achieved by "sense" technology. If a full length copy of the target gene is inserted into the genome a range of phenotypes can be obtained, some overexpressing the target gene, some under-expressing. A population of plants produced by this method may then be screened and individual phenotypes isolated.

Gene silencing can therefore be achieved by inserting into the genome of a target organism an extra copy of the target gene coding sequence which may comprise either the whole or part or be a truncated sequence and may be in sense or antisense orientation. Additionally, intron sequences which can be obtained from the genomic gene sequence may be used in the construction of suppression vectors. There have also been reports of gene silencing being achieved within organisms of both the transgene and the endogenous gene where the only sequence identity is within the promoter regions.

Although antisense and sense downregulation can result in complete silencing of the target gene, the efficiency is generally not very high. A maximum of 25% of the antisense transformants displayed complete silencing, while only about 10% of the transformants obtained with sense constructs showed some level of silencing (Smith et al. 2000 Nature 407: 319-320; Wolters and Visser, 2000 Plant Mol Biol 43: 377-386). Recently, it has been observed that the inhibition of a selected target gene within an organism is enhanced, when the gene silencing vector includes a inverted repeat of all or part of a polynucleotide region of the target gene (as suggested in WO99/61632, WO98/53083 and WO99/53050).
The inverted repeat sequence may consist of a T-DNA with one promoter driving expression of a sense copy of the cDNA together with another promoter in front of an antisense copy of the same cDNA (Chuang and Meyerowitz, 2000 Proc Natl Acad Sci USA 97: 4985-4990) or the T-DNA may contain a cDNA sequence flanked on both ends by a promoter (LaCount et al. 2000 Mol Biochem Parasit 111: 67-76), or the T-DNA may contain a promoter driving transcription of an inverted repeat of (part of) the cDNA (Hamilton et al. 1998; Smith et al. 2000 Nature 407: 319-320; Wang and Waterhouse, 2000 Wang MB, Waterhouse PM (2000) Plant Mol Biol 43: 67-82) or the promoter (Mette et al. 2000 EMBO J 19: 5194-5201) of the gene to be silenced.
Some inverted repeat constructs are reported to result in 100% of the transformants displaying silencing of the target gene (Smith et al. 2000 Nature 407: 319-320) in case of using an intron as a spacer between the repeats. The spacer fragment contributes to the stability of the perfect inverted repeat sequences, but is not required for the specificity of the silencing.

Here we demonstrate that more than 60% of potato plants transformed with a GBSSI antisense gene containing an additional, upstream inverted copy of its 5' region, exhibited substantially or even strongly reduced GBSSI activity compared to wild type plants. Only 14% of plants transformed with a similar construct, without the inverted repeat, had reduced GBSSI activity.

Although the mechanism by which the invention operates is not fully understood, we believe that creation of an inverted repeat may yield double-stranded RNA, derived from a DNA sequence showing homology to the gene to be silenced. This, in turn, is thought to trigger the degradation of the endogeneous ssRNA transcripted from the gene to be silenced Based on studies of others (Hamilton AJ et al. (1998) Plant J 15: 737-746; Stam, M (1997) Annals of Botany 79: 3-12) we suspect suppression of GBSS1 gene expression to be mainly post-transcriptional. Alternatively, Mette MF, et al.(2000) EMBO J 19: 5194-5201) describes a T-DNA containing a promoter driving transcription of an inverted repeat of (part of) the promoter (Mette et al. 2000 EMBO J 19: 5194-5201) of the gene to be silenced resulting in transcriptional silencing and promoter methylation.

The level of over-expression of a target gene in plants can be influenced by many factors. One factor is the choice of transcriptional promoters used. A range of plant compatible promoters are available including tissue specific and inducible promoters. Some of the better documented constitutive promoters include the CaMV 35S promoter and tandem arrangements of this promoter, as suggested in European patent application No. 0 342 926. Yet other factors that can be manipulated to control levels of expression are the presence of transcriptional modification factors such as introns, polyadenylation and transcription termination sites. At the translational level, other factors to consider are the ribosomal binding site and the codon bias of the gene. A translational enhancer sequence derived from the untranslated leader sequence from the mRNA of the coat protein gene of alfalfa mosaic virus coat protein gene placed between a promoter and the target gene has been shown to increase translational efficiency as suggested in US6037527.
The construct of the invention may also comprise one or more sequences that encode signal proteins, including pre-, pro- or prepro-sequences. These usually precede the sequence, such that the target gene is expressed as a fusion with the signal proteins. The signal sequence may ensure any post-translational modifications required for the formation of the mature fusion and/or may specifically direct the expressed fusion product to a desired part or organel within the plant or plant cell.

Furthermore, it has been observed that the same construct inserted at different loci on the genome can vary in the level of expression in plants. The effect is believed to be due at least in part to the position of the gene on the chromosome, i.e., individual isolates will have different expression levels. This phenomenon, referred to as "position effect" variation, is the variation in expression of the same gene in independent transformants. The use of naturally occurring DNA sequences called matrix attachment regions or scaffold attachment regions to combat this problem was proposed in U. S. Patent 5,773,689 and in WO 94/24293. WO0032800 showed that Matrix Attachment Regions could enhance expression in plant species representing both monocotyledonous and dicotyledonous plants.

In a preferred embodiment, the promoter that controls gene expression is strong and tissue-specific. Examples of known tissue-preferred promoters include the tuber-directed class I patatin promoter, (Bevan et al., (1986) Nucleic Acids Res. 14: 4625-38), the promoters associated with potato tuber GBSSI gene (Visser et al. 1991 Plant Molec. Biol. 17, 691-699), the soybean promoter of .beta.-conglycinin, also known as the 7S protein, which drives seed-directed transcription (Bray 1987 Planta 172: 364-370) and seed-directed promoters from the zein genes of maize endosperm (Pedersen et al., 1982 Cell 29: 1015-26).
Alternatively, the target gene can be under the transcriptional control of the CaMV 35S promoter. In this construct, transcriptional activity can be enhanced by a DNA fragment representing part of the CaMV 35S promoter being placed in a direct repeat tandem arrangement.

### Selection of single-copy and vector DNA-free transformants.

After transformation, the T-DNA is present in the genome of the host plant as single or multiple, copies. Furthermore, there is information that also DNA beyond the borders is sometimes integrated into the genome of the host plants. This is reported to be the case from 20-30% of the transgenic plants (Martineau, B et al. The Plant Cell 6, 1032-1033, 1994) to 75% in tobacco transformants (Kononov, M.E. et al., The Plant J. 11, 945-957). Registration authorities dealing with requests for (market) registration of transgenic plants and/or transgenic food, are of the opinion that transgenic plants with selection markers, vector DNA and multiple copies / inserts of the T-DNA should be avoided as much as possible. In the present embodiment, transformants free of backbone vector DNA are selected by PCR or Southern blotting. Alternatively the transfer of vector DNA to the plant can be inhibited by inserting a coding sequence outside the T-borders which sequence encodes a (poly)peptide that is toxic to the plant so there is counterselection for plants with superfluous vector-DNA (as suggested in WO99/01563).

### Example 1

### Optimizing the Gene Constructs for efficient inhibition of GBSSI expression

### Gene Constructs

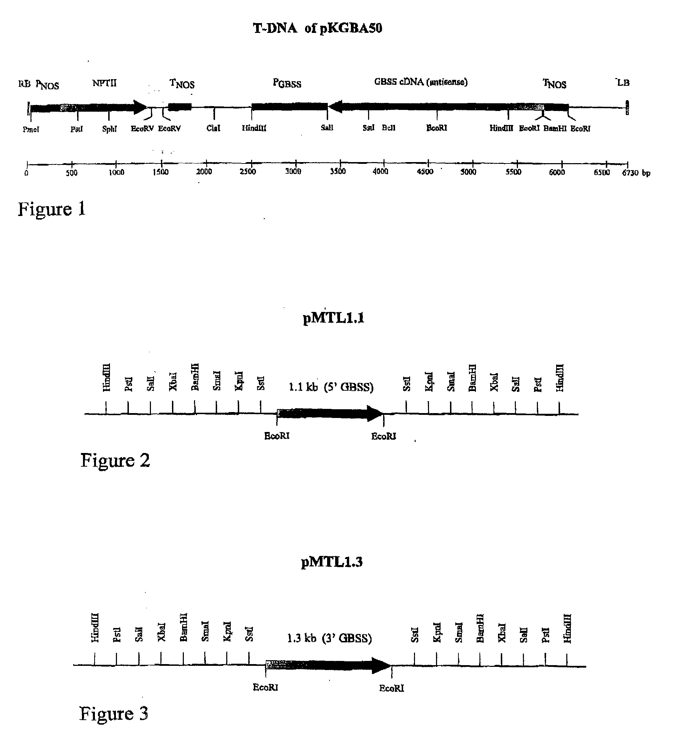

The antisense GBSSI construct pKGBA50 (Figure 1) has been described by Kuipers et al. (1995) Mol Gen Genet 246:745-755. It contains the 2.4-kb GBSSI cDNA in antisense orientation behind the GBSSI promoter in addition to selection marker gene NPTII.

Two fragments of the GBSSI cDNA, the 1.1-kb 5' part and the 1.3-kb 3' part, were cloned as EcoRI fragments in plasmids pWx1.1 and pWx1.3, respectively (Visser et al. 1991 Mol Gen Genet 225:289-296). These EcoRI fragments were subcloned into vector pMTL25 (Chambers et al. 1988 Gene 68: 139-149), resulting in plasmids pMTL1.1 and pMTL1.3, see Figures 2 and 3.

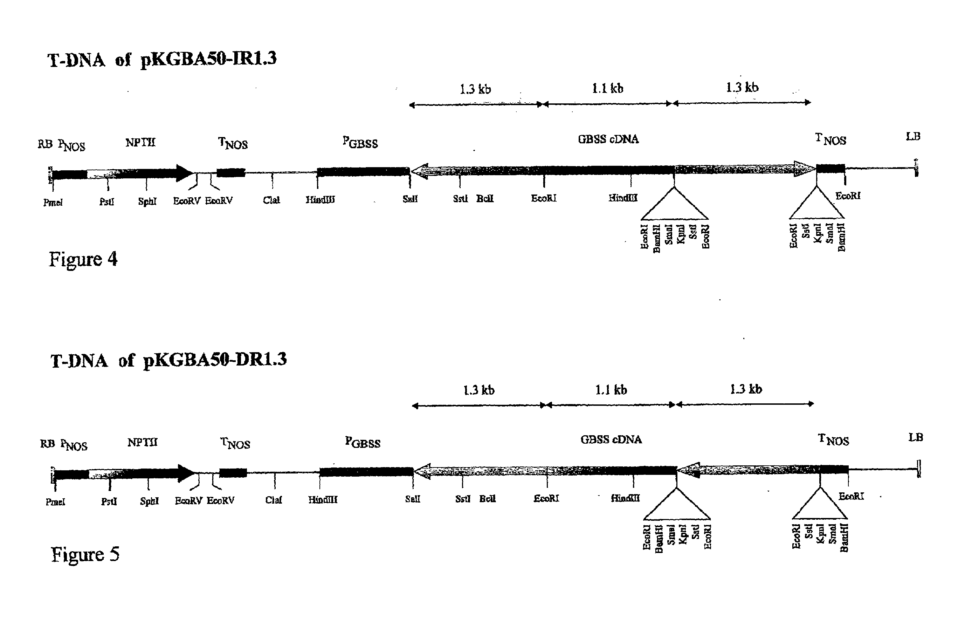

Plasmid pMTL1.3 was digested with BamHI. The ±1.3-kb BamHI fragment was cloned into BamHI-digested pKGBA50. This yielded two new binary vectors: pKGBA50-IR1.3 (Figure 4), with an inverted repeat of the 1.3-kb fragment, and pKGBA50-DR1.3 (Figure 5), containing a direct repeat of the 1.3-kb fragment.

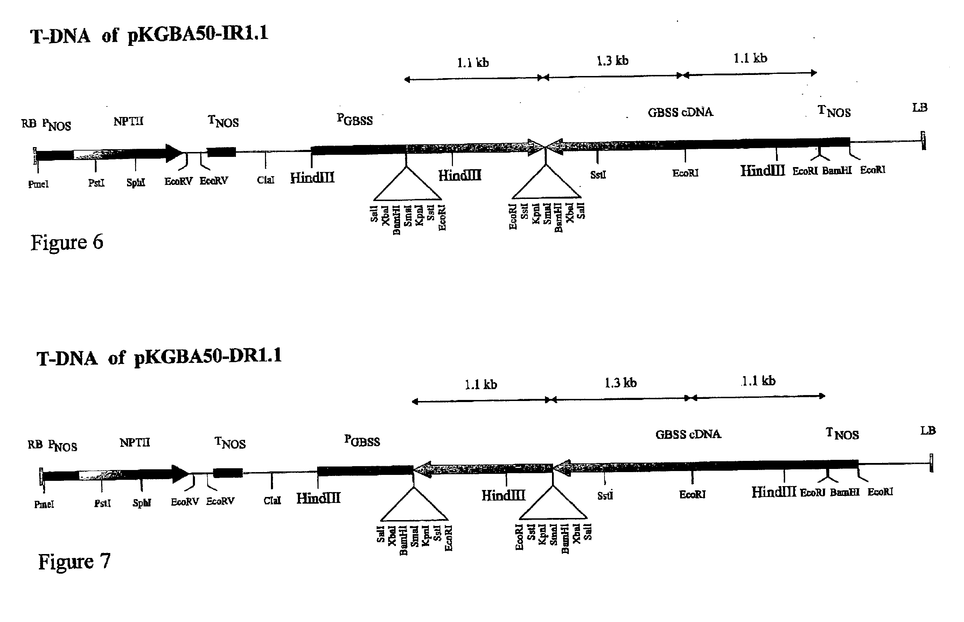

Plasmid pMTL1.1 was digested with SalI. The ±1.2-kb SalI fragment was cloned into SalI-digested pKGBA50. This yielded two additional binary vectors: pKGBA50-IR1.1 (Figure 6), containing an inverted repeat of the 1.1-kb fragment, and pKGBA50-DR1.1 (Figure 7), with a direct repeat of the 1.1-kb fragment.
All constructs were transformed into *E. coli* DH5α (GIBCO BRL). The binary vectors pKGBA50 IR1.3, pKGBA50-DR1.3, pKGBA50-IR1.1, pKGBA50-DR1.1, and pKGBA50-IR1.1 were transformed into *Agrobacterium tumefaciens* LBA4404 (pAL4404) (Hoekema et al. 1983 Nature 303:179-180) by triparental mating, using helper plasmid pRK2013 in *E. coli* HB101 (Figurski and Helinski, 1979 Proc Natl Acad Sci USA 76: 1648-1652).

### Transformation of potato

Potato cultivar 'Karnico' (Anonymous, 1994 Beschrijvende Rassenlijst voor Landbouwgewassen CPRO-DLO, Wageningen, The Netherlands, 342 pp.) had previously been transformed with antisense construct pKGBA50 (Kuipers et al. 1995 Mol Gen Genet 246:745-755). This same potato cultivar was transformed with pKGBA50-IR1.3 (construct A7), pKGBA50-DR1.3 (construct B1), pKGBA50-IR1.1 (construct C2) and pKGBA50-DR1.1 (construct D4). Internodal cuttings from *in vitro* grown plants were used for transformation by *Agrobacterium tumefaciens* co-cultivation, according to the protocol described by Visser RGF (1991) In: K Lindsey (Ed) Plant Tissue Culture Manual, Kluwer Academic Publishers, Dordrecht/Boston/London, pp. B5: 1-9. Transformants were selected on MS20 medium (Murashige and Skoog, 1962 Physiol Plant 15: 473-497) with 20 g/L sucrose, containing 100 mg/L kanamycin.

### In vitro tuberization

Shoots were transferred to pots with 50 ml solidified MS30 medium. After 3-4 weeks 20 ml of a liquid medium was added, consisting of KI medium ('knolinducerend' (= tuber inducing) medium, Duchefa) with 325 g/L sucrose and 1.75 g/L CCC (chlorocholine chloride, or cycocel.). The pots were placed in a dark growth chamber at a temperature of 18°C. After 2-6 weeks microtubers had developed on most shoots.

### Starch staining

Microtubers were cut and stained with an iodine solution (1.7% (w/v) iodine and 3.3% (w/v) potassium iodide solution), to assess the presence of amylose in the starch granules. Staining of the starch granules was inspected with a microscope.

### DNA analyses

DNA was isolated from *in vitro* shoots by a CTAB DNA isolation protocol as described by Rogers and Bendich (1988) Plant Molecular Biology Manual A6. Kluwer Academic Publ, Dordrecht, pp 1-10. PCR was performed with primers NPT3 and NPT4 (Sequence nos. 3 and 4), to check the presence of the NPTII selection marker gene. Subsequently, PCRs were performed with primers NPT1 and NPT2, trfA1 and trfA2, insB1 and insB2 (Sequence nos. 1,2,5,6,7 and 8; Wolters et al. 1998 Mol Breeding 4: 343-358), to study the presence of backbone vector DNA in the transformants.

Four µg of DNA of the transformants was digested with HindIII, fragments were separated by gel electrophoresis, and Southern blots were made. Blots were hybridized with an NPTII probe, consisting of a NPT3 + NPT4 PCR product from pBI101 (Jefferson et al. 1987 EMBO J 6: 3901-3907), to check the number of T-DNA insertions.

### Efficiency of silencing

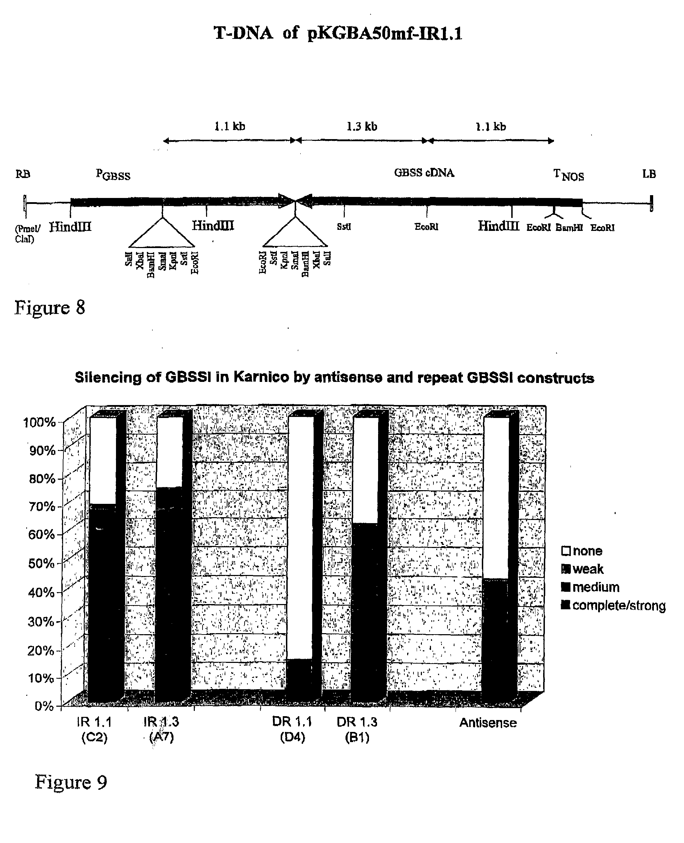

The number of transformants per construct, from which microtubers were obtained, are shown in Table 1. The iodine staining results are presented in Table 1 and Figure 9. As a reference the results obtained with antisense construct pKGBA50 are included.

Fourteen percent of the transformants obtained with antisense construct pKGBA50 showed complete inhibition of GBSSI activity, as indicated by the red colour of starch granules stained with iodine solution.

The direct repeat constructs pKGBA50-DR1.3 (B1) and pKGBA50-DR1.1 (D4) yielded a lower percentage (2-6%) of completely silenced transformants than the antisense construct. In contrast, the inverted repeat constructs pKGBA50-IR1.3 (A7) and pKGBA50-IR1.1 (C2) resulted in a higher percentage of transformants displaying complete inhibition of GBSSI, compared with the antisense construct: 20% for A7 and 62% for C2. Thus, the inverted repeat construct pKGBA50-IR1.1 (C2) was 4.5 times more efficient in completely silencing GBSSI activity than the antisense construct pKGBA50.

Inverted repeat construct C2 was much more efficient than construct A7: most (62%) of the C2 transformants showed strong or complete silencing, whereas a large proportion of the A7 transformants (47%) displayed a medium level of silencing. This suggests that either the region of the coding sequence (5' or 3' region) that is present in the inverted repeat is of importance, or that the orientation of the inverted repeat sequences relative to the promoter (antisense DNA- spacer - sense DNA vs. sense DNA - spacer - antisense DNA) is an efficiency determining factor. Similarly, there is a difference between direct repeat constructs B1 and D4; most (51%) of the B1 transformants displayed a low level of silencing, whereas a majority of the D4 transformants (85%) showed no silencing at all. Thus, the 1.1-kb region of the GBSSI cDNA was most efficient for silencing when present in an inverted repeat orientation, whereas it was least efficient when present in a direct repeat orientation. This suggests that it is important to look for the optimal sequence in order to make an inverted repeat construct that results in the highest silencing efficiency.

### Molecular analysis

DNA was isolated from 20 A7 transformants. All showed the expected NPTII PCR fragment, indicating that all contained at least one T-DNA insertion. Nine out of 20 showed the presence of non-T-DNA vector DNA, as determined by PCR of the NPTIII and trfA genes (Table 2). Thus, approximately half of the transformants did not contain backbone vector DNA.

DNA was isolated from 40 C2 transformants displaying strong to complete silencing of GBSSI. All showed the expected PCR fragment with NPTII primers. PCR analyses with backbone vector DNA primers demonstrated that 17 of these transformants contained non-T-DNA sequences, whereas 23 were negative (Table 3). Southern blot analysis of HindIII-digested DNA hybridized with an NPTII probe showed that 5 out of 20 transformants contained a single T-DNA insertion. Four of these had no backbone vector DNA. Thus, it was demonstrated that with the inverted repeat construct pKGBA50-IR1.1 it was possible to obtain completely silenced transformants with a single T-DNA insertion and no backbone vector DNA.

### Example 2

### Isolation of selection marker gene-free GBSSI-silenced transformants

### Gene constructs

Example 1 showed that the 1.1-kb region of the GBSSI cDNA was most efficient for silencing when present in an inverted repeat orientation and that it is possible with this construct (pKGBA50-IR1.1) to obtain completely silenced transformants with a single T-DNA insertion and no backbone vector DNA
In the previous example transformants were selected on kanamycin using the selectable marker gene NPTII present on construct pKGBA50-IR1.1 To isolate selection marker-free transformants the binary vector pKGBA50-IR1.1 was digested with enzymes PmeI and ClaI (see Figure 1). PmeI yields blunt ends. The ClaI sticky end was made blunt-ended by Klenow polymerase treatment, after which the vector DNA was circularized by blunt end ligation using T4 DNA ligase. This resulted in vector pKGBA50mf-IR1.1 (marker gene-free) (Figure 8).

This construct was transformed into *E. coli* DH5α (GIBCO BRL). The binary vector pKGBA50mf-IR1.1 was transformed into *Agrobacterium tumefaciens* LBA4404 (pAL4404) (Hoekema et al. 1983) by triparental mating, using helper plasmid pRK2013 in *E. coli* HB101 (Figurski and Helinski, 1979). and into *A. tumefaciens* strain Agl-0 (Lazo et al. 1991). Strain Agl-0 is known to be much more virulent than LBA4404.

### Transformation of potato and selection of transformants

Internodal cuttings from *in vitro* grown plants of potato cultivar 'Karnico' were used for transformation by *Agrobacterium tumefaciens* co-cultivation, according to the protocol described by Visser (1991).

Potato cultivar 'Karnico' was transformed with pKGBA50mf-IR1.1 in *A. tumefaciens* LBA4404 or in *A. tumefaciens* Agl-0, according to the same protocol. No selection was performed. After four weeks the first shoots were harvested and harvesting of shoots continued for about three months. No more than two regenerants per stem explant were harvested and shoots were allowed to grow on MS20 medium. After 1 to 2 weeks leaf or stem material of 8 or more independent shoots was harvested and pooled (pool size depended on the frequency of transformants expected). DNA of these pools of shoots was isolated in 96-wells microtiter plates using the Magnesil genomic DNA isolation kit purchased from Promega.

PCR analyis was performed on DNA isolated from the pools of regenerants with the primers BINMCS and GBSS-0 (Sequence nos. 9 and 10), to check for the presence of transformants. Of the PCR positive pools leaf and / or stem material of individual shoots was harvested in 96-wells microtiter plates and genomic DNA was isolated using the Magnesil genomic DNA isolation kit purchased from Promega. PCR analyis was performed on DNA isolated from the individual regenerants with the primers BINMCS and GBSS-0 (Sequence nos. 9 and 10), to check for the presence of transformants.

### In vitro tuberization

To PCR-positive shoots 20 ml of a liquid medium was added, consisting of KI medium ('knolinducerend' (= tuber inducing) medium, Duchefa) with 325 g/L sucrose and 1.75 g/L CCC (chlorocholine chloride, or cycocel.). The pots were placed in a dark growth chamber at a temperature of 18°C. After 2 to 6 weeks microtubers had developed on most shoots.

### Starch staining

Microtubers were cut and stained with an iodine solution to assess the presence of amylose in the starch granules. Staining of the starch granules was inspected with a microscope.

### DNA analyses

DNA was isolated from *in vitro* shoots by a CTAB DNA isolation protocol as described by Rogers and Bendich (1988). PCRs were performed with primers NPT1 and NPT2, trfA1 and trfA2, insB1 and insB2 (Sequence nos. 1,2,5,6,7 and 8; Wolters et al. 1998 Mol Breeding 4: 343-358), to study the presence of backbone vector DNA in the transformants.

Four µg of DNA of the transformants was digested with HindIII, fragments were separated by gel electrophoresis, and Southern blots were made. Blots were hybridized with a NOS terminator probe, to check the number of T-DNA insertions.

### Efficiency of marker-free transformation

Approximately 10,000 stem explants of potato cultivar 'Karnico' were inoculated with pKGBA50mf-IR1.1 in *A. tumefaciens* LBA4404 or in *A. tumefaciens* Agl-0. The number of shoots per construct harvested, the frequency of PCR-positive shoots obtained, and the frequency of shoots showing a phenotype are shown in Table 4.

With LBA4404 less than 0.2% of the harvested shoots were scored PCR-positive, whereas with AGL0 this percentage was more than 5%. Of these PCR-positive transformants more than 50% showed complete inhibition of GBSSI activity, as indicated by the red colour of starch granules stained with iodine solution. This percentage is comparable to the percentage of transformants displaying complete inhibition of GBSSI after selection of transformants on kanamycin, demonstrating that PCR is a reliable selection method.

Thus, transformation with a more virulent Agrobacterium strain AGL0 results in more than 30 times the number of PCR positive shoots compared to transformation with Agrobacterium strain LBA4404 and implicates the importance of optimizing the transformation process for marker-free transformation. As a result of using a highly efficient transformation protocol, an (virulent) Agrobacterium strain (like for instance AGL0), and an optimized inhibition construct pKGBA50mf-IR1.1, between 2 and 3% of the harvested shoots show complete inhibition of GBSSI expression.

### Example 3

### Effect of transformation method on the efficiency of obtaining marker-free transformants.

To determine the effect of the transformation method on the efficiency of marker-free transformation two transformation protocols were tested.
Potato plants cv. Karnico were transformed by co-cultivation of explants with *Agrobacterium tumefaciens* AGL0 containing plant transformation vector pKGBA50mf-IR1.1.

### Transformation protocol 1. 1 (Visser, 1991)

Shoot tips of potato cultivar Karnico were subcultured on media containing MS major and minor salts, 3% sucrose, 0.8% agar at pH 5.6. Cultures are grown for 4 weeks at 21 DEG C. in a 16 hour photoperiod.
Stem internodes are cut into 2 to 5mm lengths and placed on two layers of filterpaper on solid R3B media for 1 day before co-cultivation. The R3B medium used contained the salts and vitamins of MS medium (4.71 g/l) plus 3% saccharose, 2 mg/l NAA, 1 mg/l BAP and 0.8% agar, pH 5.8. The layers of filterpaper was covered with 2 ml of PACM liquid media consisting of MS (4,71 g/l) 2.0 g/l caseine hydrolysate, 3% saccharose, 1 mg/L 2,4 D and 0.5 mg/L kinetine, pH 6.5.
Co-cultivation was carried out for 5 to 10 minutes in a two-day culture of Agrobacterium before explants were blotted on filter paper to remove excess bacteria . After blotting explants were transferred back on the same petriplates and incubated for two days at 21 DEG C. in a 16 hours photoperiod.
After two days the explants were transferred to Zcvh media consisting of 4.71 g/l MS, 2.0% saccharose, 0.8% agar, 200 mg/l cefotaxime, 200 mg/l vancomycine and 1 mg/l zeatine. Cultures were transferred every two weeks to fresh Zcvh media. After four weeks the first shoots were harvested and transferred to media containing MS major and minor salts, 3% sucrose, 0.8% agar at pH 5.6. No more than two shoots per explant were harvested and harvesting continued for approximately 3 months.

### Transformation protocol 2. (Edwards et al. Plant Molec. Biol. 17: 89-100, 1991)

Shoot tips of potato cultivar Karnico were subcultured on media containing MS major and minor salts, 3% sucrose, 0.8% agar at pH 5.6. Cultures are grown for 4 weeks at 21 DEG C. in a 16 hours photoperiod.
Stem internodes are cut into 2 to 5mm lengths and placed on two layers of filterpaper on solid R3B media for 1 day before co-cultivation. The R3B medium used contained the salts and vitamins of MS medium (4.71 g/l) plus 3% saccharose, 2 mg/l NAA, 1 mg/l BAP and 0.8% agar, pH 5.8. The layers of filterpaper was covered with 2 ml of PACM liquid media consisting of MS (4,71 g/l), 2.0 g/l caseine hydrolysate, 3% saccharose, 1 mg/L 2,4 D and 0.5 mg/L kinetine, pH 5.8.
Co-cultivation was carried out for 30 minutes in a two-day culture of Agrobacterium before explants were blotted on filter paper to remove excess bacteria . After blotting explants were transferred to solid PCM media consisting of 4.71 g/l MS, 2.0% saccharose, 0.8% agar, 2 mg/l 2,4 D and 0.5 mg/l zeatine, pH 5.8 and incubated for two days in the dark at 21 DEG C. After two days the explants were transferred to PCM media containing 200 mg/l cefotaxime and incubated for four days at 21 DEG C. in a 16 hours photoperiod.
After these four days explants were transferred to solid PSM media consisting of 4.71 g/l MS, 2.0% saccharose, 0.8% agar, 2 mg/l GA₃, 1.0 mg/l zeatine, pH 5.8 and 200 mg/l cefotaxime. Cultures were transferred every three weeks to fresh PSM media containing 200 mg/l cefotaxime. After four weeks the first shoots were harvested and transferred to media containing MS major and minor salts, 3% sucrose, 0.8% agar at pH 5.6. No more than two shoots per explant were harvested and harvesting continued for approximately 3 months.

After 1 to 2 weeks leaf or stem material of 8 or more independent shoots was harvested and pooled (pool size depended on the frequency of transformants expected). DNA of these pools of shoots was isolated in 96-wells microtiter plates using the Magnesil genomic DNA isolation kit purchased from Promega.
PCR analyis was performed on DNA isolated from the pools of regenerants with the primers BINMCS and GBSS-0 (Sequence nos. 9 and 10), to check for the presence of transformants. Of the PCR positive pools leaf and / or stem material of individual shoots was harvested in 96-wells microtiter plates and genomic DNA was isolated using the Magnesil genomic DNA isolation kit purchased from Promega. PCR analyis was performed on DNA isolated from the individual regenerants with the primers BINMCS and GBSS-0 (Sequence nos. 9 and 10), to check for the presence of transformants.

### Results

Two independent transformations of 1,000 stem explants of potato cultivar 'Karnico' were carried out using transformation protocol 1 and 2. The frequency of explants from which a shoot is harvested is comparable between the two transformation protocols.
Protocol 1 resulted in 74% explantants with shoots whereas with protocol 2 67% of explantants formed one or more shoots.

The frequency of PCR-positive shoots obtained was however very different between the two methods.
With protocol 2 between 0.6 % and 1.0 % of the harvested shoots were scored PCR positive, whereas with protocol 1 this percentage was between 5.5% and 6.0%.
Thus, the use of an optimised transformation protocol may result in 5 to 10 times the number of PCR positive shoots and increases efficiency of marker-free transformation.

### Example 4

### Marker-free selection of high-lysine potato transformants

### Gene constructs

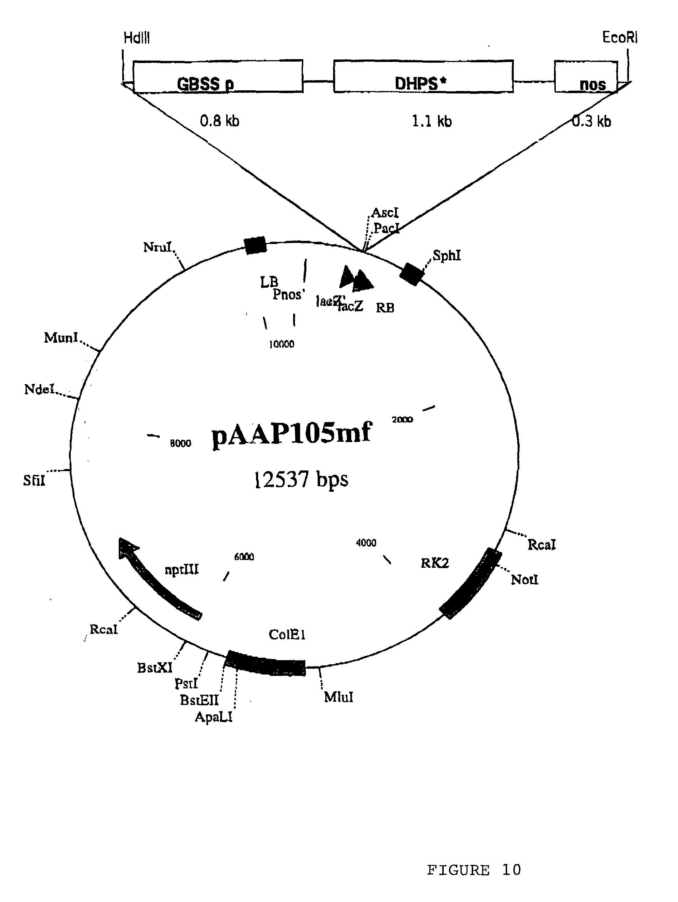

A feedback insensitive potato DHPS was created by changing the evolutionary conserved amino acid residue 134 (asparagine) into a cysteine residue, as suggested in EP99204520.
The chimeric gene containing the mutant DHPS gene was constructed by subcloning DHPS cDNA from the pTriplex vector (pAAP42) first in pCR-Script SK(+) (pAAP55) and from this vector as a XbaI-Eco RI fragment in the pBluescript SK vector digested with XbaI-EcoR (pAAP57). At the 5'end the mutated DHPS cDNA was fused to a HindIII-SalI fragment of the 800 bp long GBSSI promoter fragment. Downstream of the mutant DHPS sequence the termination signal of the nopaline synthase gene from *Agrobacterium tumefaciens* was inserted (Greve, H.D. et al. (1983) J.Mol.Appl.Genet. 1: 499-511) as an SstI-EcoRI fragment. The complete chimeric gene was subcloned into the HindIII-EcoRI sites of pBINPLUS which was first digested with ClaI and partially with RcaI and religated to remove NPTII selection marker (Van Engelen, F.A. et al. (1995) Transgenic Research 4: 288-290). This resulted in vector pAAP105mf (Figure 10). All constructs were transferred into *E. coli* DH5α (GIBCO BRL).

### Transformation of potato plants

The binary vector pAAP105mf was used for freeze-thaw transformation of *Agrobacterium tumefaciens* strain AGL0 (Höfgen, R. and Willmitzer, L. (1988) Nucl.Acids Res. 16: 9877). Transformed AGL0 was subsequently used for inoculation of potato (*Solanum tuberosum,* variety Karnico) stem explants. No selection was performed. After four weeks the first shoots were harvested and harvesting of shoots continued for about three months. No more than two regenerants per stem explant were harvested and shoots were allowed to grow on MS20 medium. After 1 to 2 weeks leaf or stem material of 8 or more independent shoots was harvested and pooled (pool size depended on the frequency of transformants expected). DNA of these pools of shoots was isolated in 96-wells microtiter plates using the Magnesil genomic DNA isolation kit from Promega.

PCR analysis was performed on DNA isolated from the pools of regenerants with the primers BINMCS and GBSS-0 (Sequence nos. 9 and 10), to check for the presence of transformants. Of the PCR positive pools leaf and / or stem material of individual shoots was harvested in 96-wells microtiter plates and genomic DNA was isolated using the Magnesil genomic DNA isolation kit from Promega. PCR analyis was performed on DNA isolated from the individual regenerants with the primers BINMCS and GBSS-0 (Sequence nos. 9 and 10), to check for the presence of transformants.

### In vitro tuberization

To PCR-positive shoots 20 ml of a liquid medium was added, consisting of KI medium. The pots were placed in a dark growth chamber at a temperature of 18°C. After 2 to 4 weeks microtubers had developed on most shoots.

### Analysis of free amino acid content in transgenic plants

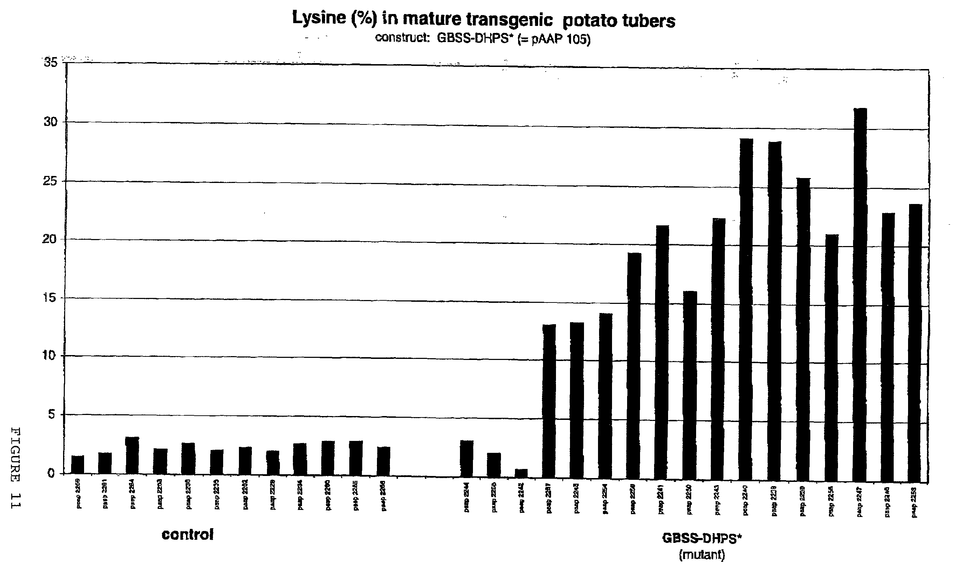

Tissue (0.5-1.0 gram) was homogenised with mortar and pestle in 2 ml 50 mM Pi-buffer (pH 7.0) containing 1 mM dithiothreitol. Nor-leucine was added as an internal standard. Free amino acids were partly purified by extraction with 5 ml of a
water:chloroform:methanol mixture (3:5:12). Water phase was collected and the organics phase was re-extracted twice. After concentration by lyophilisation to 3 ml, a 20 microl sample was analysed by HPLC using a cation-exchange column with post-column ninhydrine derivatisation of the amino acids detected at 570 and 440 nm (BIOCHROM 20, Amersham Pharmacia biotech). Figure 11 shows the lysine levels as the percentage of the total amino acids in tubers in 12 'control' untransformed potato plants and in 17 potato plants containing the mutated DHPS gene construct pAAP105mf. The lysine levels increased from 2-2.5% in the control wild type tubers to a maximum percentage of 30 in the transformed tubers, resulting in lysine being a 'bulk' amino acid in stead of a 'low level' essential amino acid .

### Example 5

### Marker-free selection of amylose-free high-lysine potato transformants

### Gene Constructs

A linker was made (5'-AGCTGCATATGAAGCTTTCTAGATCTGAATTCCATATGT-3' and
3'-CGTATACTTCGAAAGATCTAGACTTAAGGTATACATTAA-5') that contained a HindIII-compatible overhang at the 5' end, and an EcoRI-compatible overhang at the 3' end. This linker was ligated into HindIII/EcoRI-digested pUC28 plasmid (Benes et al. 1993 Gene 130: 151-152), which yielded plasmid pAAP171. Construct pAAP105, the binary vector containing the mutant DHPS gene, was digested with HindIII and EcoRI. The 2.2-kb fragment containing the GBSSI promoter-DHPS gene-NOS terminator was cloned into HindIII/EcoRI-digested pAAP171. This yielded construct pAAP172 (see Figure 12).

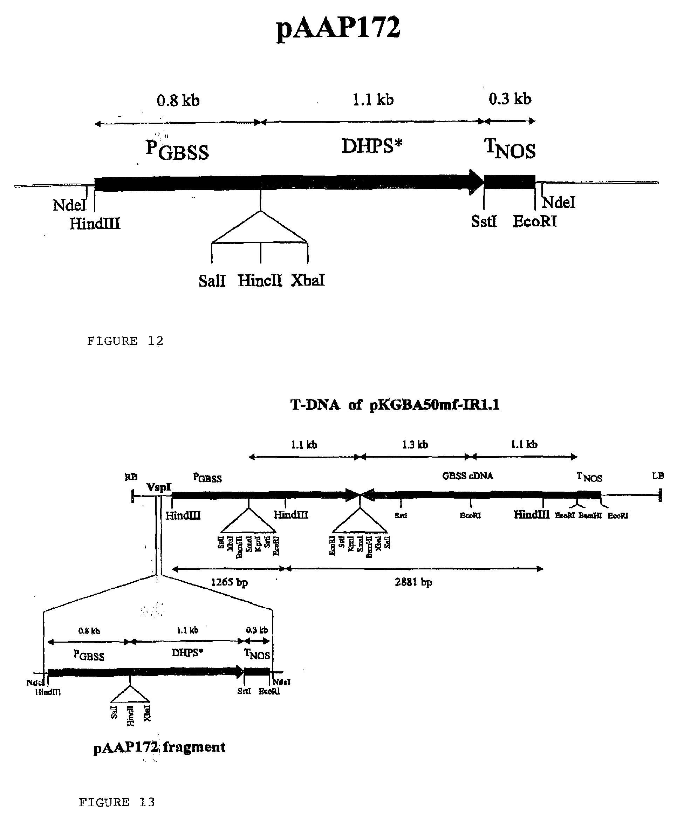

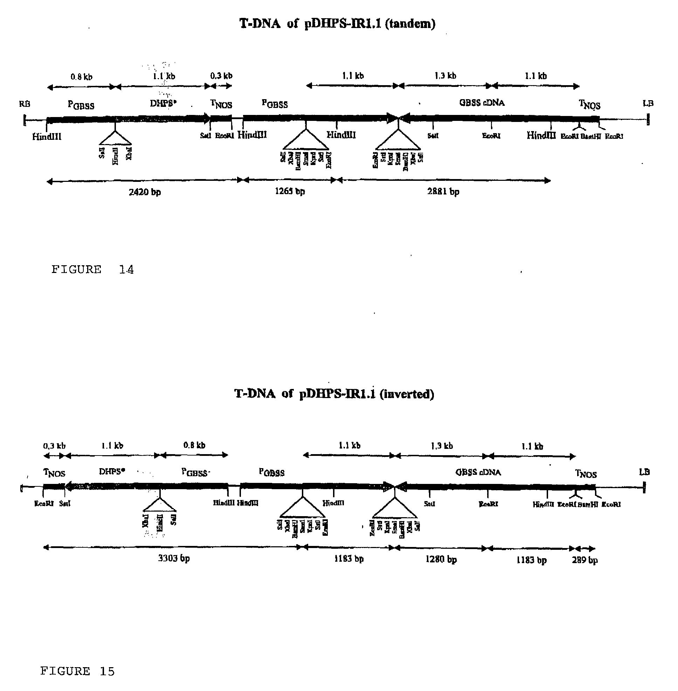

Construct pAAP172 was digested with NdeI and ScaI (a ScaI site is present in the pUC28 backbone). The digested DNA was extracted with phenol/chloroform, and ethanol-precipitated. Binary vector pKGBA50mf-IR1.1 was digested with VspI and treated with alkaline phosphatase, extracted with phenol/chloroform, and ethanol-precipitated. The 2.2-kb NdeI fragment of pAAP172 was ligated into the VspI-digested pKGBA50mf-IR1.1 vector (Figure 13), which yielded two different constructs: pDHPS-IR1.1 (tandem), in which the two GBSSI promoters point in the same direction (Figure 14), and pDHPS-IR1.1 (inverted), in which the two GBSSI promoters point in opposite directions (Figure 15). These constructs were transferred from *E. coli* DH5α into *Agrobacterium tumefaciens* Agl-0.

### Transformation of potato and selection of transformants

Internodal cuttings from *in vitro* grown plants of potato cultivar 'Karnico' were used for transformation by *Agrobacterium tumefaciens* co-cultivation, according to the protocol described by Visser (1991).

Potato cultivar 'Karnico' was transformed with pDHPS-IR1.1 (tandem) and pDHPS-IR1.1 (inverted) in *A. tumefaciens* Agl-0 according to the same protocol. No selection was performed. After four weeks the first shoots were harvested and harvesting of shoots continued for about three months. No more than two regenerants per stem explant were harvested and shoots were allowed to grow on MS20 medium. After 1 to 2 weeks leaf or stem material of 8 or more independent shoots was harvested and pooled (pool size depended on the frequency of transformants expected). DNA of these pools of shoots was isolated in 96-wells microtiter plates using the Magnesil genomic DNA isolation kit from Promega.

PCR analyis was performed on DNA isolated from the pools of regenerants with the primers BINMCS and GBSS-0 (Sequence nos. 9 and 10), to check for the presence of transformants. Of the PCR-positive pools leaf and / or stem material of individual shoots was harvested in 96-wells microtiter plates and genomic DNA was isolated using the Magnesil genomic DNA isolation kit from Promega. PCR analyis was performed on DNA isolated from the individual regenerants with the primers BINMCS and GBSS-0 (Sequence nos. 9 and 10), to check for the presence of transformants.

### In vitro tuberization

To PCR-positive shoots 20 ml of a liquid medium was added, consisting of KI medium. The pots were placed in a dark growth chamber at a temperature of 18°C. After 2 to 4 weeks microtubers had developed on most shoots.

### Starch staining

Microtubers were cut and stained with an iodine solution to assess the presence of amylose in the starch granules. Staining of the starch granules was inspected with a microscope.

### Analysis of free amino acid content in transgenic plants

Tissue (0.5-1.0 gram) was homogenised with mortar and pestle in 2 ml 50 mM Pi-buffer (pH 7.0) containing 1 mM dithiothreitol. Nor-leucine was added as an internal standard. Free amino acids were partly purified by extraction with 5 ml of a
water:chloroform:methanol mixture (3:5:12). Water phase was collected and the remaining re-extracted twice. After concentration by lyophilisation to 3 ml, a 20 microl sample was analysed by HPLC using a cation-exchange column with post-column ninhydrine derivatisation of the amino acids detected at 570 and 440 nm (BIOCHROM 20, Amersham Pharmacia biotech).

### Example 6

### Marker-free selection of amylose-free cassava transformants

The following part describes an Agrobacterium tumefaciens mediated transformation procedure to produce genetically modified cassava plants of which the starch consists predominantly of amylopectin without the help of selectable marker genes such as nptII, pat, basta, htp or others.

### Gene constructs

PCR primers (sequences nos. 12-20) were designed to amplify parts of the cassava GBSSI cDNA sequence: two nested forward and one reverse primer for each of the following three regions: the 5' part (bases 1-800), the middle part (b. 800-1500), and the 3' part (b. 1200-2000). The forward primers contained an EcoRI restriction site, whereas the reverse primers contained an XbaI restriction site.
The PCR products of each of the three parts of the GBSSI cDNA were digested with EcoRI and ligated. The obtained ligation products contained an inverted repeat of part of the cassava GBSSI cDNA, with the region between the two nested forward primers acting as a spacer. This sequence was digested with XbaI and cloned into vector pMTL24 (Chambers et al. 1988). The inverted repeat was removed from this vector by digestion with BamHI, and subsequently ligated between the potato GBSSI promoter and the NOS terminator in BamHI-digested binary vector pKGBA50mf-IR1.1 (see Figure 8). In this way three marker gene-free cassava GBSSI inverted repeat constructs were obtained. These were transformed to *Agrobacterium tumefaciens* strain Agl-0.

### Plant material and tissue culture media used

Plants of the genotype TMS60444 and Adira 4 were maintained by monthly subculture of one node cuttings on medium supplemented with Murashige and Skoog (1962, Physiol. Plant. 15: 473-497.) salts and vitamins and 40 g/l sucrose (MS4). Friable embryogenic callus (FEC) lines were initiated as follows:
- isolation of meristems or immature leaves from donor plants
- culture of meristems/leaves on MS40 supplemented with 6 mg/l NAA and 6 mg/l Picloram
- isolation of compact embryogenic tissue and culture on a medium supplemented with Gresshoff and Doy (1974, Planta 107:161-170) salts and vitamins, 60 g/l sucrose and 10 mg/l Picloram (GD6).
- isolation of FEC (small clumps of aggregated, spherical units) which were cultured on GD6 medium. FEC was maintained by a 3 weeks subculture on GD6 medium. Liquid cultures were initiated by transferring 0.5 g of FEC into flask of 200 ml with 50 ml of liquid medium supplemented with Schenk and Hildebrandt (1972, Canadian Journal of Botany 50:199-204) salts and vitamins, 60 g/l sucrose and 10 mg/l Picloram (SH6). The medium was refreshed twice a week and after 2 weeks the content of a each flask was divided over 5 new flasks. The flasks were cultured on a rotary shaker (LAB-line Instruments Inc. Model 3519) at 120 rpm.

### Infection of FEC with Agrobacterium

FEC (cultured in liquid SH6 medium or on solid gd6 medium) was sieved (mesh 1 mm) and collected. Hundred mg of FEC was spread on GD6 medium and a few drops of the Agrobacterium strain Agl-0 (derived from A281) containing the GBSSI inverted repeat construct were added to the FEC. The FEC was stored for 2 days after which the FEC was washed twice with sterile, distilled water to remove excess Agrobacterium. After the washing step the FEC was cultured in liquid SH6 medium supplemented with 400 mg/l claforan and 200 mg/l vancomycin. Again 1 week later the FEC was subcultured on GD6 medium for two more weeks after which the FEC is subcultured on maturation medium (MS4 supplemented with 1 mg/l Picloram) for 4-8 weeks (FEC transferred to fresh maturation medium every 2-3 weeks). Torpedo shaped somatic embryos were isolated from the FEC and cultured for further maturation on MS4 supplemented with 0.1 mg/l BAP. Mature somatic embryos were first cultured for two weeks in liquid and hereafter in solid germination medium (MS4+1 mg/l BAP). Plants were rooted on MS4 medium. Genetically modified plants were selected using a PCR based selection system. For this two sets of primers were developed (BINMCS against the multiple cloning site of the Agrobacterium plasmid and GBSSO against the gbss promoter). Plants which gave a positive signal with the primers were subcultured on Murashige and Skoog medium supplemented with 8% sucrose to allow starch disposition in the stems of the cassava plants (Salehuzzaman et al., 1994 Plant Science 98:53-62.). The starch of the *in vitro* thickened stems is analysed for the amylose/amylopectin ratio after staining with Lugol's solution (I2:KI). The high amylopectine plants were transferred to the greenhouse to allow formation of tuberous roots. Three months later the roots were peeled and the central cylinder of the storage root was grounded in a laboratory blender in water with a small amount of Na₂S₂ 05. The slurry was transferred for starch isolation to a Sanamat. The water starch granules solution was transferred to centrifuge tubes and centrifuged. The starch was dried at 20 degrees Celsius for 3 days. The amylopectin/amylose content was determined using the protocol described by Hovenkamp-Hermelink et al, 1988 Potato Res. 31: 241-246.

**Table 1.**

| Levels of silencing in transformants obtained with different constructs. | | | | | |
|---|---|---|---|---|---|
| Construct | No. of transformants | Level of silencing of GBSSI | | | |
| | | Complete/strong | Medium | Weak | None |
| C2 | 133 | 83 (62%) | 6 (5%) | 2 (2%) | 42 (31%) |
| A7 | 118 | 24 (20%) | 55 (47%) | 10 (8%) | 29 (25%) |
| D4 | 66 | 4 (6%) | 2 (3%) | 4 (6%) | 56 (85%) |
| B1 | 77 | 2 (2%) | 7 (9%) | 39 (51%) | 29 (38%) |
| Antisense | 144 | 20 (14%) | 20 (14%) | 22 (15%) | 82 (57%) |

**Table 2.**

| Molecular analysis of individual A7 transformants. | | | | |
|---|---|---|---|---|
| Transformant | Level of silencing | PCR fragments | | |
| | | NPTI I | NPTI II | trfA |
| A7-1 | Weak | + | - | - |
| A7-3 | None | + | - | - |
| A7-7 | Weak | + | - | - |
| A7-11 | None | + | - | - |
| A7-14 | Strong | + | + | + |
| A7-22 | Strong | + | + | + |
| A7-26 | Strong | + | + | + |
| A7-32 | Strong | + | - | - |
| A7-41 | Weak | + | - | - |
| A7-42 | Strong | + | + | + |
| A7-43 | Strong | + | + | + |
| A7-44 | Strong | + | - | - |
| A7-48 | Weak | + | - | - |
| A7-53 | Strong | + | + | + |
| A7-62 | Medium | + | + | + |
| A7-74 | Strong | + | - | - |
| A7-76 | Weak | + | - | - |
| A7-84 | Strong | + | + | - |
| A7-95 | Complete | + | + | - |
| A7-98 | Weak | + | - | - |

**Table 3.**

| Molecular analysis of individual C2 transformants. | | | | | | |
|---|---|---|---|---|---|---|
| Transformant | Level of silencing | PCR fragments | | | | No. of T-DNA bands |
| | | NPTI I | NPTI II | trfA | InsB | |
| C2-1 | Strong | + | + | ND | ND | 4 |
| C2-5 | Medium | + | + | - | - | ND |
| C2-6 | Weak | + | - | - | - | ND |
| C2-7 | Complete | + | + | ND | ND | >5 |
| C2-8 | Complete | + | + | ND | ND | >5 |
| C2-10 | Strong | + | + | ND | ND | 3 |
| C2-11 | Complete | + | + | + | ND | >5 |
| C2-14 | Complete | + | - | - | ND | 3 |
| C2-15 | Complete | + | - | - | ND | 4 |
| C2-16 | Medium | + | - | - | ND | 2 |
| C2-17 | Complete | + | ? | + | ND | 3 |
| C2-20 | Complete | + | - | - | ND | 1 |
| C2-25 | Complete | + | + | ND | ND | 2 |
| C2-29 | Complete | + | - | - | ND | 1 |
| C2-30 | Complete | + | + | ND | ND | >4 |
| C2-33 | Strong | + | - | - | - | ND |
| C2-38 | Strong | + | - | - | ND | 1 |
| C2-41 | Strong | + | - | - | - | ND |
| C2-43 | Strong | + | + | + | - | ND |
| C2-47 | Complete | + | - | + | - | ND |
| C2-65 | Complete | + | + | ND | ND | >4 |
| C2-66 | Complete | + | - | - | ND | 2 |
| C2-70 | Strong | + | - | - | - | ND |
| C2-71 | Complete | + | - | - | ND | 2 |
| C2-75 | Strong | + | - | - | - | ND |
| C2-80 | Strong | + | - | - | - | ND |
| C2-83 | Strong | + | + | ND | ND | 1 |
| C2-85 | Strong | + | + | + | + | ND |
| C2-86 | Medium | + | - | - | - | ND |
| C2-95 | Strong | + | - | - | - | ND |
| C2-112 | Strong | + | + | + | - | ND |
| C2-114 | Complete | + | - | - | ND | 1 |
| C2-116 | Strong | + | + | ND | ND | 4 |
| C2-151 | Strong | + | - | - | - | ND |
| C2-152 | Strong | + | - | - | - | ND |
| C2-156 | Complete | + | - | - | - | ND |
| C2-167 | Strong | + | + | - | + | ND |
| C2-168 | Strong | + | - | - | - | ND |
| C2-170 | Strong | + | - | - | - | ND |
| C2-180 | Strong | + | - | - | - | ND |
| ND = not determined | | | | | | |

**Table 4.**

| Efficient marker-free silencing of the GBSS I gene potato with an inverted repeat construct. | | | |
|---|---|---|---|
| A.tum strain | No. of shoots harvested | No. of PCR-positive plants (% of total shoots) | No. of amylose-free plants (% of total shoots) |
| LBA4404 | 2504 | 4 (0.16%) | 2 (0.08%) |
| AGLO | 1508 | 79 (5.2%) | 37 (2.5%) |
| | | | |

### Primer Sequences

Sequence identity no. 1 (NPT1):
5'-TCC ACC TTA TCG GCA ATG AA-3'

Sequence identity no. 2 (NPT2):
5'-CGG CAG TGA GAG CAG AGA TA-3'

Sequence identity no. 3 (NPT3):
5'-TCG GCT ATG ACT GGG CAC AAC AGA-3'

Sequence identity no. 4 (NPT4):
5'-AAG AAG GCG ATA GAA GGC GAT GCG-3'

Sequence identity no. 5 (trfA1):
5'-TTC TCC TCG TGC TCG TAA AC-3'

Sequence identity no. 6 (trfA2):
5'-GGT CGC TGG TAT TCG TGC-3'

Sequence identity no. 7 (insB1):
5'-GCG CTA TCT CTG CTC TCA CT-3'

Sequence identity no. 8 (insB2):
5'-AAC GGC CTC ACC CCA AAA A-3'

Sequence identity no. 9 (BINMCS):
5'-GCA CCC CAG GCT TTA CAC TT-3'

Sequence identity no. 10 (GBSS-0):
5'-TAC CGC TAC CAC TTG ACA TTC-3'

Sequence no. 11:
5' AGCT GCATATGAAGCTTTCTAGATCTGAATTCCATATGT 3'
3' CGTATACTTCGAAAGATCTAGACTTAAGGTATACATTAA 5'

Sequence no. 12:
Primer 5CASGBF1: 5'-TAG AAT TCA CCA GCG GAA CCT ATT TT-3'

Sequence no. 13:
Primer 5CASGBF2: 5'-ATG AAT TCG GAC CCA AAC TAT CAC TC-3'

Sequence no. 14:
Primer 5CASGBR1: 5'-TGT CTA GAA TGG AAG CAG AGC AGT GT-3'

Sequence no. 15:
Primer MCASGBF1:5'-CAG AAT TCA AGC CAT TTA CCA ACC TA-3'

Sequence no. 16:
Primer MCASGBF2: 5'-ATG AAT TCT ATG AGA AGC CCG TGA AG-3'

Sequence no. 17:
Primer MCASGBR1: 5'-CAT CTA GAG AAC CAG CAT AAA GTC AG-3'

Sequence no. 18:

Primer 3CASGBF1: 5'-TAG AAT TCG GCT TCA TTG GTA GAT TA-3'

Sequence no. 19:
Primer 3CASGBF2: 5'-TAG AAT TCA TTG AGC ATC TGG AGG TT-3'

Sequence no. 20:
Primer 3CASGBR1: 5'-TAT CTA GAC CAT TCA CCC TTC ACA AA-3'

## Claims

1. A recombinant nucleic acid comprising a T-DNA construct allowing for transfer of said construct into the genome of a plant cell, said construct provided with a foreign nucleic acid that is free of nucleic acid encoding a selective marker.

2. A nucleic acid according to claim 1 wherein said T-DNA construct comprises at least one T-DNA border.

3. A nucleic acid according to claim 1 or 2 wherein said foreign nucleic acid is flanked by T-DNA border repeats.

4. A nucleic acid according to anyone of claims 1 to 3 wherein said T-DNA is derived from the Ti plasmid of an Agrobacterium spp.

5. A nucleic acid according to claim 4 wherein said Agrobacterium comprises A. tumefaciens.

6. A nucleic acid according to anyone of claims 1 to 5 wherein said foreign nucleic acid allows for regulation of the expression of a target gene in said genome.

7. A nucleic acid according to claim 6 wherein said regulation comprises downregulation.

8. A nucleic acid according to claim 7 wherein said foreign nucleic acid includes a inverted repeat of at least part of a polynucleotide region of said target gene.

9. A nucleic acid according to claim 6, 7 or 8 wherein said target gene encodes a granule-bound starch synthase (GBSSI) enzyme.

10. A nucleic acid according to anyone of claims 1 to 5 wherein said foreign nucleic acid allows for expression of a heterologous polypeptide in said plant cell.

11. A nucleic acid according to claim 10 wherein said heterologous polypeptide comprises an enzyme.

12. A nucleic acid according to claim 11 wherein said enzyme comprises a, preferably feedback-insensitive, dihydrodipicolinate synthase (DHPS)

13. A vector or plasmid comprising a nucleic acid according to anyone of claims 1 to 12.

14. A host cell comprising a nucleic acid according to anyone of claims 1 to 12 or a vector according to claim 13.

15. A host cell according to claim 14 which comprises an Agrobacterium.

16. A host cell according to claim 15 which comprises a substantially virulent Agrobacterium.

17. A host cell according to claim 15 or 16 wherein said Agrobacterium comprises A. tumefaciens.

18. A host cell according to claim 17 wherein said host cell comprises A. tumefaciens A281 or cell derived thereof.

19. A plant cell comprising a nucleic acid according to anyone of claims 1 to 12.

20. A plant, or part thereof, derived from a plant cell according to claim 19.

21. A tuberous plant, or part thereof, according to claim 20.

22. A plant, or part thereof, according to claim 21 which is selected from the group of potato or cassave plants.

23. A method for transforming a plant cell comprising providing a plant cell with a nucleic acid according to anyone of claims 1 to 12.

24. A method according to claim 23 further comprising culturing said cell under essentially no selective pressure derived from a selective medium.

25. A method according to claim 23 or 24 further comprising testing at least a part of the progeny of said cell for the presence or absence of at least a functional part of a nucleic acid according to anyone of claim 1 to 12.

26. A method according to anyone of claims 23 to 25 further comprising testing at least a part of the progeny of said cell for the presence or absence of undesired vector nucleic acid.

27. A plant cell obtainable with a method according to anyone of claims 23 to 26.

28. A plant, or part thereof, derived from a plant cell according to claim 27.

29. A tuberous plant, or part thereof, according to claim 28.

30. A plant, or part thereof, according to claim 29 which is selected from the group of potato or cassave plants.
